# EUROPEAN PATENT APPLICATION

(11) **EP 0 820 772 A1**
(43) Date of publication of application: **28.01.1998**
(21) Application number: 95923615.9
(22) Date of filing: 27.04.1995
(51) Int. Cl.: A61K 38/21, A61K 31/435, A61K 47/44, A61K 9/127, A61K 9/50

(54) **IMMUNOMODULATING MEDICINAL AGENT**

(30) Priority: 23.03.1995 RU 95103740
(71) Applicant: Travkin, Oleg Viktorovich, St. Peterburg, 195297 (RU); Genkin, Dmitry Dmitrievich, S-Peterburg, 197198 (RU)
(72) Inventor: Travkin, Oleg Viktorovich, St. Peterburg, 195297 (RU); Genkin, Dmitry Dmitrievich, S-Peterburg, 197198 (RU)
(74) Representative: Zellentin, Rüdiger
(86) International application number: RU9500079
(87) International publication number: WO9607423

(57) **Abstract**

The invention relates to medicine and veterinary science, specifically to immunomodulating medicinal preparations based on hydrophobic interferon inducers used in anti-viral therapy. The problem addressed by the invention is to broaden the range of effective uses, improve potency, reduce toxicity and simplify production of the agent in question. A novelty is the use of complex ethers of 10-methylene carboxy-9-acridone as a low molecular weight interferon inducer in conjunction with a pharmaceutically acceptable carrier, and the selection of substances used to ensure solubility in water and for microencapsulation in the case of a lipid carrier.

## Description

### Sphere of technology.

The present invention relates to medicine and veterinary science, and specifically to immunomodulant medicaments based on hydrophobic derivatives of interferon inducers used in antiviral therapy.

### Previous level of technology.

The following immunomodulant drugs based on interferon inducers used in antiviral therapy (see, for example, [1] - [8]) are commonly known:
- natural high-molecular inducers (polyribonucleic acids), for example larifan (ridostin) [1];
- synthetic high-molecular inducers (polyribonucleic acids), for example polyguanyl (poly (G)), polyinoxyl(poly (I)) and other acids [2];
- natural (vegetable) polyphenols, for example gossinol [3];
- synthetic analogues of vegetable polyphenols, for example megasin [4];
- synthetic low-molecular inducers based on:
   a) 10-acridonacetic acid or its sodium salt [5];
   b) 3.6 - bis(heteroaminapoxy)acridines, specifically on 3.6 - bis(2-dimethylaminetoxy)acridine [6].

The above-named medicaments have a determinate prophylactic and therapeutic effect. They are used in aqueous solutions, ointments or in other pharmaceutically acceptable vehicles.

It is also known that the above-named medicaments have a number of drawbacks. Specifically, natural and synthetic polyribonucleic acids are highly toxic and are rapidly destroyed by nucleases in the patient's organism. Natural and synthetic polyphenols are characterized by relatively low interferon-inducing activity and by toxicity. Synthetic low-molecular interferon inducers based on 10 - acridonacetic acid or its sodium salt, though highly effective and possessing low toxicity, are hydrophylic; the latter property impedes the penetration of these drugs through the lipid layer of cell membranes, consequently reducing their effect on target cells in the immune system and thus impeding their penetration of histohematic barriers (including hemato-encephalitic barriers). Furthermore, the hydrophylic character of these drugs leads to their being rapidly excreted from the organism, necessitating repeat injections of the drug in order to achieve the desired therapeutic effect.
Synthetic low-molecular inducers based on 3.6 - bis(2-dimethylaminetoxy)acridine, and specifically on 3.6 - bis(2-dimethylaminetoxy)-4.5-dichlorine-2-nitroacridine, are hydrophobic and do not have the drawbacks of the above-named inducer based on 10 - acridonacetic acid or its sodium salt. This medicament is, in its nature and in the way in which it acts upon the cells of the immune system, the closest to the medicament which is the subject of the present application (hereinafter called "the proposed drug"); for this reason it has been chosen as the prototype.

The prototype is described in greater detail in USA patent Number 4314061, M 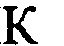 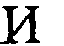 C07D 413/14, H 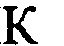 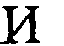 544-80 (applied for: 01.08.77; published: 02.02.82; authors: K.C. Murdock, M.R. Damiani, F.E. Durr).

Like other analogues, the prototype possesses, besides its great merits, a number of weak points:
- it has a comparatively narrow field of effective action (it is mainly effective against herpes simplex - 1 virus);
- it has high toxicity;
- it is insufficiently effective especially when administered locally;
- it is difficult to produce.

### Description of the invention.

The purpose of the present invention is to reduce the drawbacks indicated above, i.e. to produce a medicament which has a broader field of effective action, is more effective, is less toxic, and is simpler to manufacture.

The goals defined above have been met by making significant changes to an already existing medicament containing a low-molecular interferon inducer and a pharmaceutically acceptable vehicle. The nature of these changes is that ether of 10 - methylencarboxylat-9-acridone ether has been selected as the interferon inducer.

Moreover, where use is made of an aqueous vehicle, solubility may be enhanced by adding to the solution pH-stabilizing additives, for example hydroxides of alkaline and alkaline-earth metals, in quantities equal to that of the ether.

Moreover, where use is made of a lipid vehicle, the solution may be microcapsulated to increase its effectiveness.

The choice of inducer indicated above is based on the results of investigations carried out by the authors of the present invention, which investigations have led to the discovery of new physical and mechanical properties of complex ethers of 10 - methylencarboxylat-9-acridone. The most fundamental of the latter properties are: the high pharmacological activity of such ethers; new pharmacological properties of such ethers; the ability of such ethers to dissolve in water in the presence of hydroxides of alkaline and alkaline-earth metals; the ability of such ethers to incorporate into artificial lipid layers at pH values which are neutral or close to neutral. It is these characteristics and properties of complex ethers of 10 - methylencarboxylat-9-acridone -- properties which were unknown to the authors from the sources of information available to them -- that permitted the realization of the goals stated above.

Medicines based on complex ethers of 10 - methylencarboxylat-9-acridone contain complex ether and stabilizing additives in quantities optimal for the desired pharmaceutical effect, dissolved in apyrogenic water (or other pharmaceutically acceptable vehicle) or incorporated into artificial lipid structures (or other pharmaceutically acceptable vehicles). The medicinal structure given in the present application made possible the production of a range of biologically active and pharmaceutically acceptable medicines suitable for parenteral, peroral, inhalational, epidermal and intranasal use. In other words, a new immunomodulant drug has been created on the basis of pharmaceutically acceptable complexes of ethers of 10 - methylencarboxylat-9-acridone and macromolecular vehicles. The macromolecular interferonogenic complexes thus obtained possess the following characteristics: increased efficiency of interferon induction; a broader range of antiviral activity; decreased frequency of side effects; enhanced pharmaceutical properties. Use has been made of aqueous solutions and lipid smectic mesophases (liposomes) containing ethers of 10 - methylencarboxylat-9-acridone. In particular, use has been made of the said ethers in combination with amphiphilic lipid molecules. The latter initially form a complex solution in acceptable organic solvents; subsequently, as the organic solvent is gradually replaced with a pharmaceutically acceptable aqueous solution, lipid smectic mesophases (liposomes) are formed comprising artificial spherical membranes of 60 - 7000 nanometers in size, consisting of one or more layers in which ethers of 10 - methylencarboxylat-9-acridone are bonded to lipids by non-covalent (proton and hydrophobic) bonds.

The proposed drug is produced in ways of which the following is an example. A mixture of lipids (0.5 - 0.9 eqv.) and complex ether of 10 - methylencarboxylat-9-acridone (0.3 - 0.1 eqv.) is directly or indirectly dissolved in an acceptable organic solvent (chlorinated hydrocarbons, simple aliphatic ethers, alcohols, or other acceptable solvents). The given proportion of complex ether to lipids is optimal since increasing the proportion of complex ether to more than 0.1 - 0.3 eqv. (depending on the structure of the ether radical) decreases the stability of the complex formed leading to crystallization of the complex ether of 10 - methylencarboxylat-9-acridone. Decreasing the proportion of complex ether results in a decrease in interferon-inducing activity and to a decline in the pharmaceutical acceptability of the complex. The organic solvent is replaced with an aqueous solvent either by preliminary elimination of the solvent followed by hydration of the dry substance, or by elimination of the organic phase from an emulsion containing organic solvent and aqueous phase. Moreover, procedures for replacement of organic solvent with an aqueous phase should be carried out under a higher temperature than the temperature of phase transition of the most refractory of the lipids present in the mixture. The volume of organic phase is determined in accordance with the solubility of components in the acceptable solvent; the volume of aqueous solution is determined in accordance with the number of components used for the complex synthesis.

The complexes thus obtained were analyzed using scan and transmission electron microscopy, laserscopy, flow cytofluorometry, high-resolution liquid chromatography and column chromatography on POLY-PREP hydrophobic columns. Such analysis revealed that the yield of homogenous stable liposomes is not less than 95%, and that not less than 89% of ether used is integrated in stable form into the liposomes.

The feasibility of producing the proposed medicaments is illustrated by the following practical examples.

### EXAMPLE 1.

100 g. of ethyl ether of 10 - methylencarboxylat-9-acridone is mixed with 500 ml. of apyrogenic water; 1.05 eqv. of sodium hydroxide is added. The mixture thus obtained is kept at a temperature of 90 - 95°C for 1 hour. During this time 80 ml. of solvent are distilled and cooled to room temperature. 400 ml. of apyrogenic water is added to the mixture thus obtained. The mixture is then filtered. The content of ethyl ether of 10 - methylencarboxylat-9-acridone in the filtrate is determined by spectrofluorometry. Apyrogenic water is added until the desirable concentration has been reached. Citric acid (approximately 1.5 g.) is added until pH = 7.75 has been reached. The medicine thus obtained is filtered through a bacterial filter and poured into bottles, which are then hermetically sealed and sterilized. The above process gives a medicine ready for parenteral introduction and with a yield of approximately 98% (i.e. ethyl ether of 10 - methylencarboxylat-9-acridone). The parenteral medicine described above may contain pH- stabilizing additives such as substituted phenols, carbonic acids, salts of weak alkalis and strong acids.

### EXAMPLE 2.

500 mg. of lipids (dipalmitoylphosphatidylcholin, phosphatidylserin in a molar proportion of 10 : 0.1) and 100 mg. of lauryl ether of 10 - methylencarboxylat-9-acridone are dissolved in 500 ml. of chloroform at 22°C and placed in a thick-walled round-bottomed 4-litre flask. The flask is placed on a rotor vaporizer and the solvent is eliminated at 65°C. A thin semi-transparent film of a greenish hue forms on the walls of the flask. The flask is put into a drying chamber equipped with a nitrogen trap, and is further dried in a vacuum for 1 hour. 10 ml. of twice-distilled deionized water are added to the flask. After the flask has been ventilated with helium, the film is rehydrated by vigorous shaking at 65°C for 1 hour. As a result, 10 ml. of thick gel with a greenish hue is formed.

The gel is diluted using 90 ml. of phosphate buffer (pH=7.4, 22°C) and subjected to ultrasound at 44kHz 30 times for 30 seconds at 1-minute intervals. The resulting suspension is compressed (twice, consecutively) under pressure of nitrogen through polycarbonate membranes the diameter of whose pores is first 500 and then 100 nanometers. An opalescent suspension of a greenish hue is formed; this comprises an aqueous suspension of small single-layer liposomes of 80 - 130 nanometers in size, which liposomes contain 500 mg. of lipids and 100 mg. of lauryl ether of 10 - methylencarboxylat-9-acridone. In the presence of a stabilizer (threchalose), the suspension is frozen in liquid nitrogen, following which it is subjected to lyophilic drying over the course of 24 hours. The powder thus obtained is microcapsulated with an acid-resistant covering. Microcapsules containing 500 mg. of lipids and 100 mg. of lauryl ether of 10 - methylencarboxylat-9-acridone are pressed into tablets, each of which contains 20 mg. of acridone derivative and 100 mg. of lipid.

### EXAMPLE 3.

500 mg. of lipids (phosphatidylcholin, phospatidylethanolamin, cholesterol in a molar proportion of 1 : 0.001 : 0.5) are dissolved in 400 ml. of diethyl ether at a temperature of 22°C. 50 mg. of ethyl ether of 10 - methylencarboxylat-9-acridone is dissolved in 1 ml. of dimethylsulfoxide at 60°C before being cooled to 22°C and added in drops to 400 ml. of the solution of lipids in diethyl ether. The resulting solution is intensively mixed. 100 ml. of double-distilled water is added to the solution. The diphase system thus obtained is treated with ultrasound until the border dividing the two phases disappears and a disperse system (emulsion) forms. The organic phase is eliminated in a rotor vaporizer at a temperature exceeding the temperature of phase transition of the most refractory phospholipid (60°C) among those present in the mixture; during the vaporizing process the vacuum is gradually increased to 0.1 tor. 100 ml. of thick light-green gel is obtained. After freezing in liquid nitrogen, the gel is subjected to lyophilic drying, giving 550 mg. of light-green powder. The powder is mixed with 10 ml. of phosphate buffer (pH = 7.0, 22°C) to give a light-green suspension comprising an aqueous suspension of polystratified liposomes of 400 - 800 nanometers in size, which liposomes contain 500 mg. of lipids and 50 mg. of ethyl ether of 10 - methylencarboxylat-9-acridone. Subsequently: as in Example 2.

### Industrial application

The enclosed tables 1, 2, 3, and 4 show the results of trials of the proposed medicaments in comparison with analogues, the prototype and a control group.

Table 1 shows the effectiveness of the proposed drug for treatment of infectious processes with a viral origin (in comparison with other drugs and with the prototype).

Table 2 shows the antitumorous effectiveness of the proposed drug (in comparison with other drugs and with the prototype).

Table 3 shows the effectiveness of use of the proposed drug for treatment of developing leukosis L 1210.

Table 4 shows the comparative effectiveness of different forms of the proposed drug in treatment of the influenza virus A/Aichi/2/68(H3N2).

It follows from Table 1 that the proposed drug has, of the drugs tested in this trial, the broadest range of effective application in treatment of infectious processes of viral origin; moreover, it is several times more effective than the prototype, and is even more effective in comparison with other widely used drugs. Table 2 demonstrates that the effectiveness of the proposed drug in treatment of tumorous growths is several times higher that of the prototype and is comparable to that of conventional antitumorous drugs. From Table 3 it follows that use of the proposed drug in the treatment of leukosis L1210 is twice as effective as use of the prototype in reducing the percentage of tumour cells in the blood. Table 4 shows that the index of antiviral activity of a soluble form of the proposed drug is higher than that for a suspension of the drug, whilst the index of antiviral activity of liposomal forms is higher than that for soluble forms of the drug (on the basis of a single injection of the drug 1 hour before infection with the A/A ichi / 2/68 (H3N2) virus).

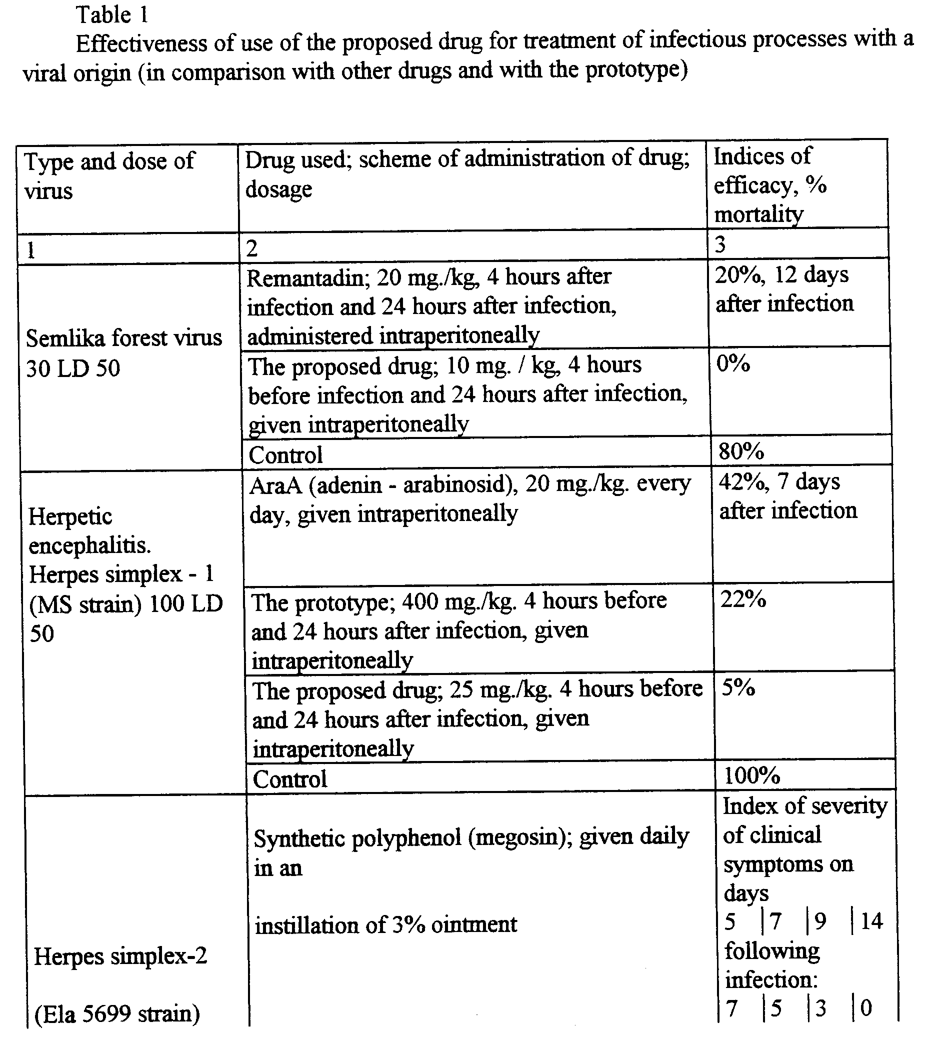

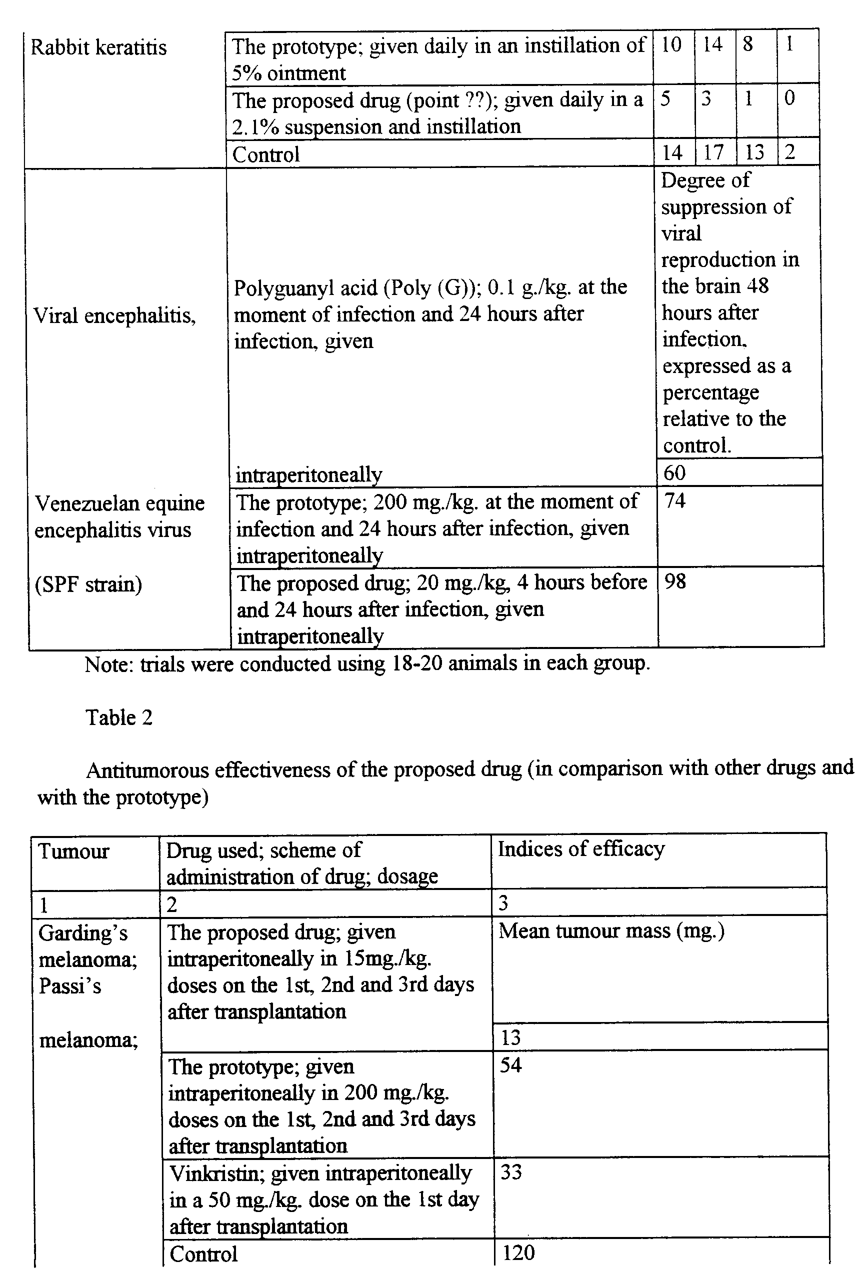

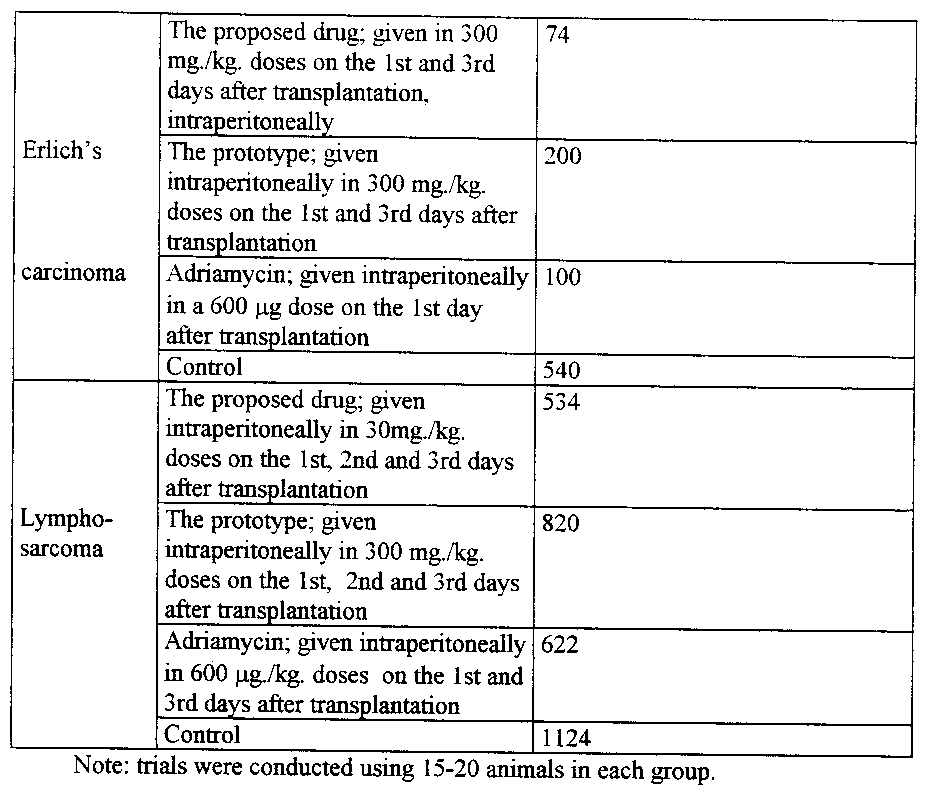

**Table 3**

| Effectiveness of use of the proposed drug for treatment of developing leukosis L 1210. | |
|---|---|
| Drug used; scheme of administration of drug; dosage | Percentage of blastular cells in peripheral blood |
| 1 | 2 |
| The proposed drug; given orally in 30 mg./kg. doses on the 1st, 2nd and 3rd days after transplantation | 18 |
| The prototype; given orally in 400 mg./kg. doses on the 1st, 2nd and 3rd days after transplantation | 40 |
| Control | 96 |
| Note: trials were conducted using 20-26 animals in each group. | |

**Table 4**

| Comparative effectiveness of different forms of the proposed drug in treatment of the influenza virus A/Aichi/2/68(H3N2). | | | | |
|---|---|---|---|---|
| Form of drug | Number of animals | Dosage of virus | Index of protection on the 7th day (%) | Index of protection on the 12th day (%) |
| suspension | 12 | 1 LD 50 | 78 | 68 |
| | 13 | 10 LD 50 | | |
| aqueous solution | 12 | 1 LD 50 | 98 | 91 |
| | 14 | 10 LD 50 | 79 | 72 |
| liposome form | 12 | 1 LD 50 | 100 | 89 |
| | 14 | 10 LD 50 | 83 | 79 |

In addition to specific results obtained from the experiments conducted above, constant observation was made of side effects such as irritation of the skin and mucous membranes and changes in the cardiovascular, digestive and excretory systems. The toxicity (therapeutic index) of the proposed drug was 100-500, whilst that of the prototype was 10-15.

From the examples given above of production of the proposed drug it is clear that the said drug is relatively simple to manufacture; its manufacture is at least one and a half times less labour-intensive than that of the prototype.

Thus it is our opinion that the proposed drug is a new drug; displays a level of invention (albeit not immediately obvious); is suitable for industrial manufacture; and, in making available a highly effective medicament for parenteral, enteral, external and other uses, successfully solves a problem of importance to mankind.

References
1. State Register of Pharmaceuticals Permitted for Use in Medical Practice and Industrial Production. Moscow, The Ministry of Health of the USSR, 1990.
2. Gordon G. Minks, M.A. The interferon renaissance: molecular aspects of induction and action. Microbiol. Rev, vol. 45, p.244, 1981.
3. Hudson J.B. Antiviral compounds from plants. CRC Press, 1990.
4. F.I. Ershov. "Interferon", in the book "General and Particular Virology", Moscow, Meditsina, pp.323-341, 1982.
5. USA Patent Number 3681360, "Antiviral substitute acridones". Applied for: 09.04.71; published: 01.06.72.
6. USA Patent Number 4314061, M 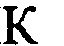 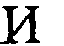 C 07D 413/14, H 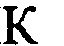 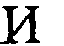 544-80, "Some 3.6-bis(heteroaminoalcoxi) acridines". K. C. Murdock, R. Damiani, F.E. Durr. Applied for: 01.08.77; published: 02.02.82.
7. I. Gamage, A. Swarha, W. Gordon. "Structural- activity relationship for substituted 9-ox-9,10-dihidracridine-4-acetic-acid" in J. Anti-Cancer Drug. Des, pp.403-414, 7(5), 1992.
8. J. Tareporewala, B. Zrach et al. "Synthesis and structure- active relationship of anti-inflammatory 9.10-dihidro- -oxo-2-acridinealkanoic acids and 4(2- carboxylatphenyl)" in J. Pharm. Sci. pp.173-178, 79(2), 1990.

## Claims

1. An immunomodulant drug containing a hydrophobic interferon inducer and a pharmaceutically acceptable vehicle *the distinguishing characteristic of which drug is* that in it ether 10-methylencarboxylat-9-acridone is used as the interferon inducer:

2. A drug meeting the specification given in point 1, *the distinguishing characteristic of which drug* is that when use is made of an aqueous vehicle to the solution are added pH-stabilizing additives (for example a citrated buffer) and hydroxides of alkaline and alkaline-earth metals in amounts equal to the ether present in the solution;

3. A drug meeting the specification given in point 1, *the distinguishing characteristic of which drug* is that when use is made of a lipid vehicle the preparation is microcapsulated.
